# EUROPEAN PATENT APPLICATION

(11) **EP 2 704 044 A2**
(43) Date of publication of application: **05.03.2014**
(21) Application number: 13178549.5
(22) Date of filing: 30.07.2013
(51) Int. Cl.: G06F 19/00

(54) **Apparatus and method for providing medical support**

(30) Priority: 30.08.2012 JP 2012190376
(71) Applicant: Fujifilm Corporation, Minato-ku Tokyo (JP)
(72) Inventor: Ohta, Yasunori, Kanagawa (JP)
(74) Representative: Metzler, Volker

(57) **Abstract**

A decline notification receiving section (35) of a management server (25) of a medical support apparatus (17) receives a decline notification from an external support terminal (13) of a medical support staff (decliner) who declines or stops participating in medical support. A substitute medical staff requesting section (36) accesses a medical support staff database (25a) in which data of medical support staffs is stored, creates a candidate list (89) of substitute medical staffs capable of providing the medical support in place of the decliner, and transmits the candidate list to the decliner's external support terminal. The decliner selects a substitute medical staff from the candidate list and transmits data of the selected substitute medical staff to a substitute medical staff data receiving section (37). A terminal management section (33) starts managing an external substitute terminal (14) of the selected substitute medical staff as a terminal for providing the medical support to an in-hospital terminal (16).

## Description

### BACKGROUND OF THE INVENTION

### 1. Field of the invention

The present invention relates to an apparatus and a method for providing medical support using network communications.

### 2. Description Related to the Prior Art

There are many clinics only with generalist physicians in rural areas, for example. It is difficult for a generalist physician (hereinafter simply referred to as generalist) to treat a patient who needs diagnosis and treatment of a specialist physician (hereinafter simply referred to as specialist) with a specialty.

In such cases, it is known to provide medical support using a medical support apparatus (the so-called cyber hospital system) (for example, see Japanese Patent Laid-Open Publication No. 2004-280807). In the cyber hospital system, an in-hospital terminal located inside a medical institution of a primary care physician, being the generalist, is connected to an external terminal through a network. The external terminal is used by a medical support staff such as a specialist of another medical institution. The medical support is provided through the in-hospital terminal and the external terminal. Here, the medical support includes diagnosis performed by the medical support staff based on medical information of a patient sent from the primary care physician and transmission of the result of the diagnosis and the medical support staff's advice on treatment of the patient to the primary care physician.

Normally, a medical support staff belongs to a medical institution such as a hospital. The medical support staff cannot provide or perform the medical support when occupied by tasks in his/her medical institution or the disease in question is outside his/her specialty. The medical support staff may need to stop the medical support when he/she is suddenly caught up in his/her own tasks during the medical support. If the medical support staff declines or stops the medical support, the treatment is interrupted. There is a possibility that a substitute medical staff cannot be found soon.

### SUMMARY OF THE INVENTION

An object of the present invention is to provide an apparatus and a method for providing medical support which facilitates finding a substitute medical staff even if a medical support staff declines to participate in the medical support.

To achieve the above and other objects, a medical support apparatus comprises a delivery means, a decline notification receiving means, a substitute medical staff requesting means, a substitute medical staff data receiving means, and a terminal management means. The medical support apparatus is connected to an in-hospital terminal, at least one external support terminal, and at least one external substitute terminal through a network. The in-hospital terminal is used by a primary care physician that requests the medical support. The at least one external support terminal is used by a medical support staff selected by the primary care physician. The at least one external substitute terminal is used by a substitute medical staff. The delivery means transmits a medical support request to the each external support terminal. The medical support request is transmitted to the delivery means from the in-hospital terminal. The decline notification receiving means receives a decline notification for declining participation in the medical support. The decline notification is transmitted from a first external support terminal. The first external support terminal is one of the at least one external support terminal. The first external support terminal is used by a decliner that declines the medical support request. The substitute medical staff requesting means transmits a substitute medical staff request to the first external support terminal to request the decliner to recommend the substitute medical staff. The substitute medical staff data receiving means receives information of the substitute medical staff, recommended by the decliner, from the first external support terminal. The terminal management means manages the in-hospital terminal and the external support terminal as a terminal group for performing the medical support. The terminal management means allows a first external substitute terminal used by the substitute medical staff, out of the at least one external substitute terminal, to join the terminal group in place of the first external support terminal when the substitute medical staff provides the medical support in place of the decliner. The delivery means transmits information pertaining to the medical support within the terminal group.

It is preferable that the medical support staff is selected by the primary care physician from among candidates stored in a medical support staff candidate database. The medical support staff candidate database stores information pertaining to medical qualification and specialty of the candidate and information pertaining to the external support terminal.

It is preferable that the substitute medical staff requesting means retrieves at least one of the candidates stored in the medical support staff candidate database to create a candidate list for the substitute medical staff. The retrieved candidate has the same medical qualification and the same specialty as those of the decliner. The substitute medical staff requesting means transmits the candidate list and the substitute medical staff request to the first external support terminal. The decliner uses the candidate list to recommend the substitute medical staff.

It is preferable that the terminal management means notifies the primary care physician's in-hospital terminal that the medical support staff declines the participation in the medical support and the substitute medical staff participates in the medical support.

It is preferable that when the decliner recommends an unregistered medical support staff not listed in the candidate list as the substitute medical staff, information of the unregistered medical support staff is transmitted from the first external support terminal to the substitute medical staff data receiving means.

It is preferable that the substitute medical staff data receiving means transmits the information of the unregistered medical support staff to the in-hospital terminal to ask for the primary care physician's approval. The substitute medical staff data receiving means permits the unregistered medical support staff as the substitute medical staff when receiving notification of the primary care physician's approval from the in-hospital terminal. The terminal management means manages the first external substitute terminal as one of terminals of the terminal group in place of the first external support terminal. The first external substitute terminal is used by the substitute medical staff approved by the primary care physician.

It is preferable that the substitute medical staff data receiving means permits the unregistered medical support staff as the substitute medical staff without the primary care physician's approval when medical qualification of the unregistered medical support staff is other than a predetermined medical qualification. The terminal management means manages the first external substitute terminal as one of terminals of the terminal group, in place of the fist external support terminal. The first external substitute terminal is used by the substitute medical staff permitted by the substitute medical staff data receiving means.

A medical support method of the present invention is used for providing the medical support through a medical support system. In the medical support system, an in-hospital terminal, at least one external support terminal, at least one external substitute terminal, and a medical support apparatus are connected to each other through a network. The in-hospital terminal is used by a primary care physician that requests the medical support. The at least one external support terminal is used by a medical support staff selected by the primary care physician. The at least one external substitute terminal is used by a substitute medical staff. The medical support is provided through the medical support apparatus. The method comprises a medical support request transmitting step, a medical support request delivering step, a decline notification transmitting step, a substitute medical staff request transmitting step, a substitute medical staff information transmitting step, and a managing step, and information delivering step. The medical support request transmitting step transmits a medical support request from the in-hospital terminal to the medical support apparatus. The medical support request delivering step delivers the medical support request from the medical support apparatus to the each external support terminal. The decline notification transmitting step transmits a decline notification for declining participation in the medical support from a first external support terminal to the medical support apparatus. The first external support terminal is one of the at least one external support terminal. The first external support terminal is used by a decliner that declines the medical support request. The substitute medical staff request transmitting step transmits a substitute medical staff request from the medical support apparatus to the first external support terminal to request the decliner to recommend the substitute medical staff. The substitute medical staff information transmitting step transmits information of the substitute medical staff recommended by the decliner from the first external support terminal to the medical support apparatus. The managing step manages the in-hospital terminal and the external support terminal as a terminal group for performing the medical support. The first external substitute terminal used by the substitute medical staff, out of the at least one external substitute terminal, joins the terminal group in place of the first external support terminal when the substitute medical staff provides the medical support in place of the decliner. The information delivering step delivers information pertaining to the medical support within the terminal group.

According to the present invention, when the medical support staff (decliner) declines to participate in the medical support, a substitute medical staff that performs the medical support in place of the decliner is selected or appointed. The treatment is continued without interruption. The substitute medical staff is selected from the previously registered candidate list. Thereby, the substitute medical staff is selected quickly. The decliner can recommend a person not on the candidate list, so that the optimum substitute medical staff is surely found. The new substitute medical staff requires the primary care physician's approval. This improves mutual reliability within a medical support team centered on the primary care physician.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Fig. 1 is a schematic view illustrating a medical support system;
Fig. 2 is an explanatory view illustrating a medical support staff database;
Fig. 3 is a functional block diagram illustrating how a management server functions when it receives a medical support request;
Fig. 4 is a functional block diagram illustrating how the management server functions when it receives data of a substitute medical staff selected from a candidate list;
Fig. 5 is a functional block diagram illustrating how the management server functions when it registers a new substitute medical staff;
Fig. 6 is a block diagram illustrating functions of an external support terminal;
Fig. 7 is an explanatory view illustrating a medical support request screen of an in-hospital terminal;
Fig. 8 is an explanatory view illustrating a medical support request receiving screen of the external support terminal;
Fig. 9 is an explanatory view illustrating a medical support screen of the external support terminal;
Fig. 10 is an explanatory view illustrating the medical support screen on which an examination image is displayed;
Fig. 11 is an explanatory view illustrating the medical support screen on which a remark input section is displayed;
Fig. 12 is an explanatory view illustrating a substitute medical staff selection screen of the external support terminal;
Fig. 13 is an explanatory view illustrating a new substitute medical staff registration screen of the external support terminal;
Fig. 14 is an explanatory view illustrating the medical support screen of the in-hospital terminal;
Fig. 15 is a flowchart illustrating steps for requesting medical support;
Fig. 16 is a flowchart illustrating steps for providing the medical support;
Fig. 17 is a flowchart illustrating steps for the medical support staff to decline to participate in the medical support; and
Fig. 18 is a flowchart illustrating steps for permitting a new substitute medical staff to join a medical support team without primary care physician's approval.

### DESCRIPTION OF THE PREFERRED EMBODIMENTS

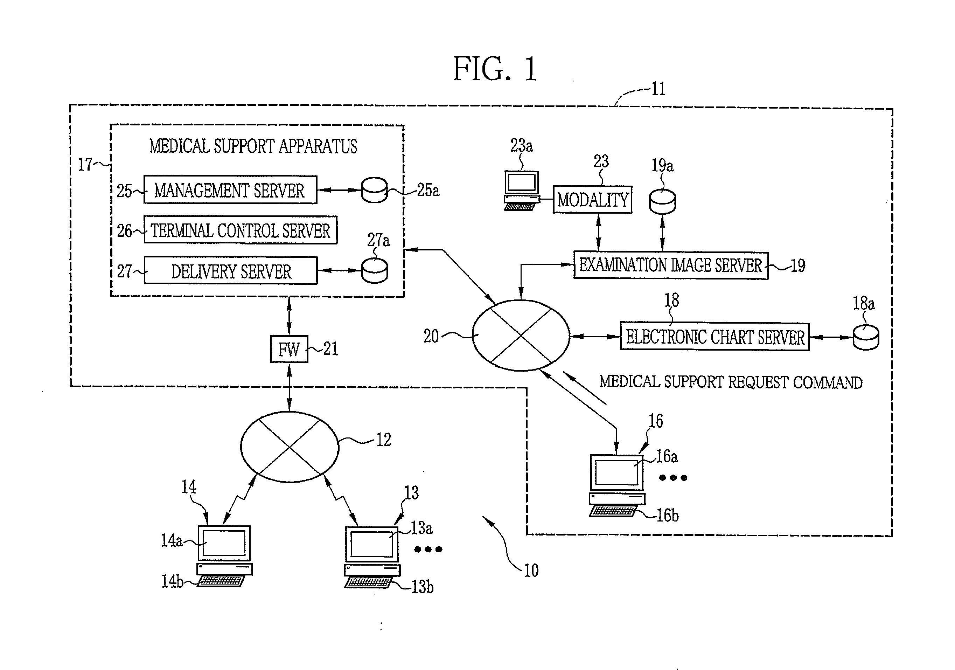

In Fig. 1, a medical support system 10 of the present invention comprises an in-hospital subsystem 11, an external support terminal 13, and an external substitute terminal 14. The in-hospital subsystem 11 is built in a hospital which needs medical support. The external support terminal 13 and the external substitute terminal 14 are connected to the in-hospital subsystem 11 through a network 12. The external support terminal 13 is used by an external medical support staff (hereinafter simply referred to as the medical support staff) who provides medical support from outside of the hospital. The external substitute terminal 14 is used by an external substitute medical support staff (hereinafter simply referred to as the substitute medical staff) who provides the medical support, in place of the medical support staff, from outside of the hospital.

The medical support in this embodiment includes diagnosis and treatment performed by the medical support staff in place of a primary care physician and advice on the diagnosis and treatment from the medical support staff to the primary care physician. Namely, the medical support staff works together with the primary care physician to provide optimum medical services. The medical support staff has specialized knowledge and experience in a specific medical field and capable of providing the medical support in his/her specialty. For example, the medical support staff is a doctor, a nurse, a radiation technologist, or a lab technologist. The medical support staff may be a hospital clerk. The medical support staffs constitute a medical support team. Note that only one medical support staff may be included in the medical support team, depending on a disease or injury of a patient.

Any type of network 12 can be used as long as it connects the in-hospital subsystem 11, the external support terminal 13, and the external substitute terminal 14 in a communicable manner. The network 12 is, for example, the Internet, an exclusive line, a mobile phone line, a public wireless LAN (Local Area Network), or a combination thereof. Note that it is preferable to connect the in-hospital subsystem 11, the external support terminal 13, and the external substitute terminal 14 through VPN (Virtual Private Network) to prevent information leak.

The in-hospital subsystem 11 comprises an in-hospital terminal 16, a medical support apparatus (in-hospital medical support system) 17, an electronic chart server 18, an examination image server 19, an in-hospital LAN 20, a firewall 21, and the like. The firewall 21 is provided between the medical support apparatus 17 and the network 12. The in-hospital LAN 20 is a network built in the hospital using the Internet technology. The in-hospital LAN connects the in-hospital terminal 16, the medical support apparatus 17, and the servers 18 and 19 in a communicable manner. The firewall 21 permits communications only between the external support terminal 13, the external substitute terminal 14, and the medical support apparatus 17 through the network 12. The firewall 21 blocks unauthorized access to the medical support apparatus 17 and other apparatuses connected to the in-hospital LAN 20 to prevent the information leak and alteration of data.

The in-hospital terminals 16 are disposed in examination rooms and treatment rooms of various departments, for example, surgery and internal medicine, in a hospital. The in-hospital terminal 16 is a personal computer on which an application is installed. The application includes a program for electronic charts, a program for requesting examinations, and a program for medical support. A monitor 16a of the in-hospital terminal 16 displays an electronic chart, a medical image, and various screens for the medical support, for example, a medical support request screen for requesting the medical support. A keyboard 16b and a mouse (not shown) of the in-hospital terminal 16 are used for various operations pertaining to the medical support, for example, referring to or inputting an electronic chart, requesting an examination such as X-ray imaging, and referring to medical images. Data of a medical support staff may be deleted or newly registered by accessing the medical support apparatus 17 through the in-hospital terminal 16.

The electronic chart server 18 stores an electronic chart, being medical information, in an electronic chart database 18a and provides the data as necessary. The electronic chart is composed of patient data, treatment data, and the like. The patient data includes the name, the date of birth, gender, the weight, the height, and the like of a patient. The electronic chart server 18 stores the patient data as basic data record in the electronic chart database 18a. The basic data record also includes a patient ID assigned to each patient. The basic data record is identified using the patient ID. A new basic data record is created for a new patient (patient with no patient ID) and stored in the electronic chart database 18a by inputting the patient data using a terminal (not shown) for new registration.

The electronic chart server 18 stores the treatment data, inputted to the electronic chart through the in-hospital terminal 16, as treatment data record in the electronic chart database 18a. The treatment data includes findings and prescriptions, for example. A registration ID and the patient ID are stored in the treatment data record. The registration ID is issued from a registration terminal (not shown) when the treatment is registered. The treatment data includes an ID (primary care physician ID) of a primary care physician. When the treatment data includes an examination image, its ID (examination image ID) is also stored in the treatment data record.

A modality 23, such as a CT device or an MRI device is connected to the examination image server 19. The examination image server 19 stores an examination image, such as a CT image obtained from the modality 23, in an examination image database 19a and provides the examination image as necessary. The examination image server 19 receives an examination request from the in-hospital terminal 16, and sends a command based on the examination request to an operation terminal 23a of the modality 23. An examination image of the patient taken by a technologist using the modality 23 is stored as an examination image record in the examination image database 19a. The examination image record includes an examination ID, an imaging time, a technologist ID assigned to the technologist who took the examination image, the registration ID, and the patient ID, in addition to the examination image.

When the medical support is started, the medical support apparatus 17, for example, notifies the electronic chart server 18 and the examination image server 19 of the registration ID and the patient ID of the patient, being the subject of the medical support. Upon receipt of the notification, the electronic chart server 18 sends the patient data and treatment data, corresponding to the registration ID, to the medical support apparatus 17. The examination image server 19 sends the examination image and its associated data, corresponding to the patient ID, to the medical support apparatus 17. When the content of the electronic chart corresponding to the registration ID is updated, the electronic chart server 18 sends the updated content to the medical support apparatus 17. When a new examination image corresponding to the patient ID of the patient, being the subject of the medical support, is taken, the examination image server 19 sends the new examination image to the medical support apparatus 17. Various types of medical information sent to the medical support apparatus 17 are transmitted to the medical support staff to whom the medical support is requested. Note that the medical information including the electronic chart, the examination image, and the like may be transmitted manually by the primary care physician.

The medical support apparatus 17 comprises a management server 25, a terminal control server 26, a delivery server 27, and the like. The management server 25 is a personal computer on which an application such as the program for medical support is installed. The management server 25 registers a medical support staff, cancels the registration of a medical support staff, authenticates a medical support staff, receives a decline notification, for declining participation in the medical support, from a medical support staff, and registers a substitute medical staff, for example. Declining the participation in the medical support includes declining a medical support request and stopping the medical support before the medical support is completed.

A medical support staff database 25a, in which data pertaining to medical support staff(s) is stored, is connected to the management server 25. By accessing the management server 25 through the in-hospital terminal 16 or a management terminal (not shown), a new medical support staff is registered, data of an existing medical support staff is changed, or the registration of an existing medical support staff is canceled. When a new medical support staff is registered, the management server 25 creates a new medical support staff record and stores it in the medical support staff database 25a. When the registration of an existing medical support staff is cancelled, the management server 25 deletes the corresponding medical support staff record from the medical support staff database 25a.

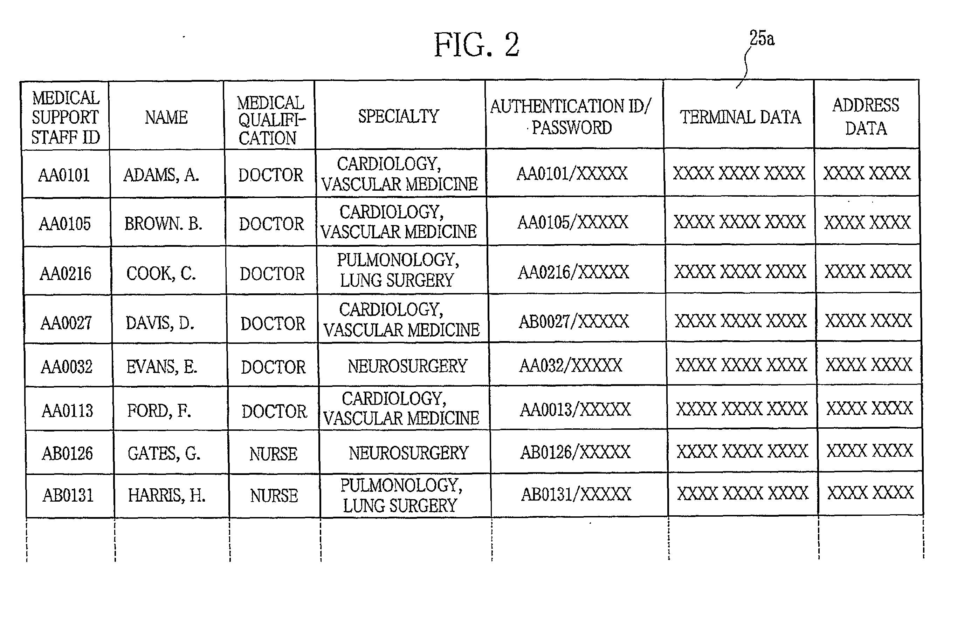

As shown in Fig. 2, a medical support staff ID for identifying a medical support staff, attribute information of the medical support staff, an ID (authentication ID) and a password for authentication, data (terminal data) of the terminal in use, address data of the terminal, and the like are stored in the medical support staff record in the medical support staff database 25a. The attribute information includes the name of the medical support staff, data of medical qualification (or job title) of the medical support staff such as doctor, nurse, or technologist, and data (specialty data) of his/her specialty. The specialty data may be "cardiology and vascular medicine (heart and blood vessels)" "neurosurgery (brain surgery)", "pulmonology (respiratory medicine) and lung surgery", or the like. The terminal data is used to identify the external support terminal 13 and the external substitute terminal 14. For example, a MAC address assigned to a network interface of the external support terminal 13 or the external substitute terminal 14 is used as the terminal data. The address data is an IP address, a mail address, or the like of each of the external support terminal 13 and the external substitute terminal 14 to which data is sent. The medical support staff records with different terminal data and address data may be stored per medical support staff so as to make two or more external support terminals 13 available for one medical support staff. In the case where two or more medical support staffs use a single external support terminal, the external support terminal 13 and the external substitute terminal 14 may be one and the same.

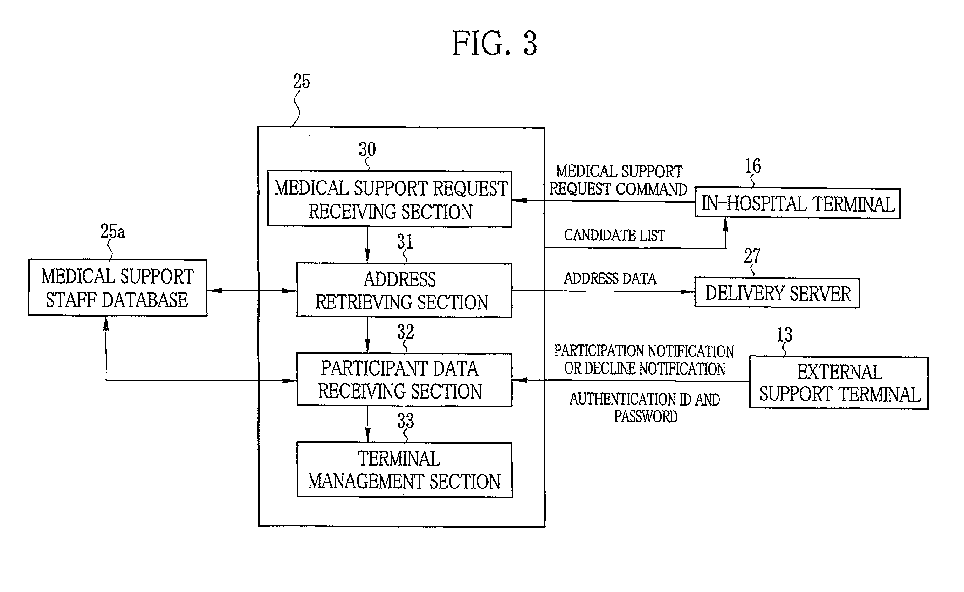

As shown in Fig. 3, the management server 25 functions as a medical support request receiving section 30, an address retrieving section 31, a participant data receiving section 32, and a terminal management section 33 when the program for medical support is started. The medical support request receiving section 30 receives a medical support request command from the in-hospital terminal 16. The medical support request command instructs the transmission of a medical support request to a medical support staff appointed or selected by the primary care physician. The address retrieving section 31 accesses the medical support staff database 25a and retrieves the address data of the external support terminal 13 corresponding to the destination (medical support staff) selected by the primary care physician. The retrieved address data of the medical support staff is sent to the delivery server (delivery section) 27. The delivery server 27 sends the medical support request to the external support terminal 13.

The participant data receiving section 32 receives a participation notification or a decline notification from the external support terminal 13. In addition to the participation notification, the authentication ID and the password of the medical support staff and the terminal data of the external support terminal 13 in use are sent to the participant data receiving section 32. The participant data receiving section 32 authenticates the sender of the participation notification as the medical support staff, using the authentication ID, the password, and the terminal data. The sender of the participation notification is accepted as the valid medical support staff when the authentication ID, the password, and the terminal data match the registered data. The terminal management section 33 manages the external support terminal 13 of the valid medical support staff as the external support terminal used for the medical support.

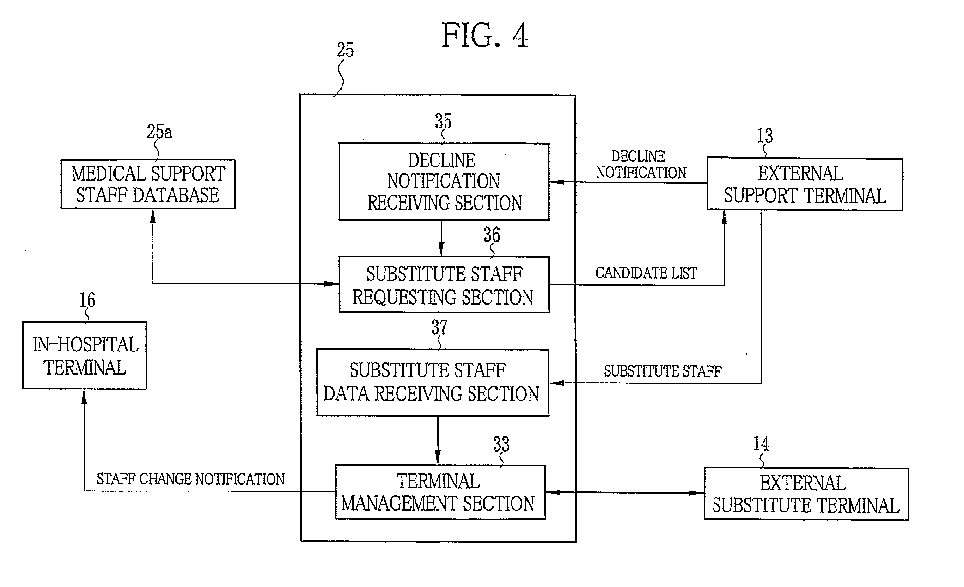

Normally, a medical support staff belongs to a medical institution. The medical support staff needs to decline or refuse the medical support request or stop the medical support when he/she is occupied by tasks in his/her medical institution or when he/she lacks experience or knowledge of the treatment for the disease or injury of the patient, for example. As shown in Fig. 4, the management server 25 functions as a decline notification receiving section 35, a substitute medical staff requesting section 36, a substitute medical staff data receiving section 37, and the terminal management section 33 when the medical support staff declines or stops the medical support.

The decline notification receiving section 35 receives the decline notification from the external support terminal 13. The decline notification notifies that the medical support staff using the external support terminal 13 declines the request (medical support request) for the medical support or stops the medical support. The substitute medical staff requesting section 36 sends a request (substitute medical staff request) for a substitute medical staff to the external support terminal 13 to request the medical support staff (hereinafter may be referred to as the decliner), who declines the participation in the medical support, to recommend a substitute medical staff. The substitute medical staff performs the medical support in place of the medical support staff (decliner) who declines the participation in the medical support. The substitute medical staff data receiving section 37 receives data of a substitute medical staff from the external support terminal 13 operated by the decliner. When the substitute medical staff is determined, the terminal management section 33 starts managing the external substitute terminal 14 of the substitute medical staff as a terminal used for the medical support.

The substitute medical staff requesting section 36 transmits a list (candidate list) of candidates for a substitute medical staff together with the substitute medical staff request to the external support terminal 13 used by the decliner. To create the candidate list, the substitute medical staff requesting section 36 retrieves medical support staffs, with the same medical qualification or job title and the same specialty as those of the decliner, from the medical support staff database 25a. The substitute medical staff data receiving section 37 receives data pertaining to the substitute medical staff, selected or recommended by the decliner based on the candidate list, from the external support terminal 13.

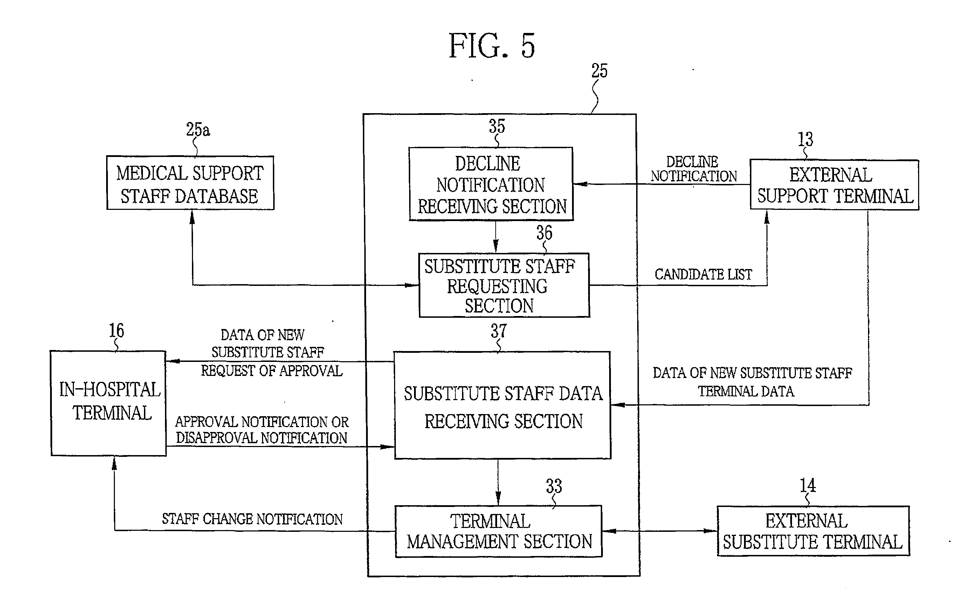

Alternatively or in addition to the data pertaining to the substitute medical staff selected from the candidate list, the substitute medical staff data receiving section 37 can receive data pertaining to a new substitute medical staff, not registered on the medical support staff database 25a, from the external support terminal 13. As shown in Fig. 5, when the substitute medical staff data receiving section 37 receives the data pertaining to a new substitute medical staff recommended by the decliner from the external support terminal 13, the data of the new substitute medical staff is transmitted to the primary care physician's in-hospital terminal 16 through the delivery server 27, and the substitute medical staff data receiving section 37 asks for primary care physician's approval of the new substitute medical staff. The approval is necessary because the new substitute medical staff is not a medical support staff selected based on experience and ability and registered on the medical support staff database 25a, so that it is necessary to determine whether the substitute medical staff is suitable for the medical support.

When the primary care physician approves the new substitute medical staff, the substitute medical staff data receiving section 37 allows the terminal management section 33 to manage the new substitute medical staff's external substitute terminal 14 as the external support terminal used for the medical support for the in-hospital terminal 16. Note that, when the new substitute medical staff is not approved, the substitute medical staff data receiving section 37 prevents the terminal management section 33 from managing the external substitute terminal 14 used by the new substitute medical staff.

After the substitute medical staff data receiving section 37 receives the data of the substitute medical staff, the terminal management section 33 allows the delivery server 27 to transmit a staff change notification to the primary care physician's in-hospital terminal 16. The staff change notification notifies that the current medical support staff stops the medical support and a substitute medical staff takes over the medical support, for example. The terminal management section 33 manages the in-hospital terminal 16 used by the primary care physician, the external support terminal 13 used by the medical support staff who accepted to participate in the medical support, and/or the external substitute terminal 14 as a terminal group for performing the medical support.

The delivery server 27 delivers or distributes the medical support request, medical information, and an opinion within the terminal group for performing the medical support. When the medical support is requested, the delivery server 27 delivers the medical support request to the medical support staff's external support terminal 13 based on the address data from the address retrieving section 31. The medical support request includes medical support request data comprising a patient ID of the patient, being the subject of the medical support, a registration ID and registration time of the case of the patient, a medical support request comment inputted by the primary care physician, examination image(s) taken with the modality 23 between the acceptance of the patient and the transmission of the medical support request command, and the medical information such as contents of the electronic chart of the case of the patient. The delivery server 27 retrieves the content of the electronic chart and the examination image(s) necessary for the medical support request data from the electronic chart server 18 and the examination image server 19, using, for example, the registration ID and the examination image ID as keys.

After the medical support is started, the delivery server 27 receives the medical information. The medical information includes an opinion or remark sent from the primary care physician's in-hospital terminal 16, the external support terminal 13, or the external substitute terminal 14, the updated content of the electronic chart from the electronic chart server 18, and new examination image (s) from the examination image server 19. The delivery server 27 delivers or distributes the medical information and its associated data to each of the in-hospital terminal 16, the external support terminal 13, and/or the external substitute terminal 14. The destination is determined based on management data of the terminal management section 33. Thereby, the medical support team is formed. For example, the medical support team is composed of two persons, the primary care physician and the medical support staff, when the primary care physician selects one medical support staff. If the medical support staff declines, the primary care physician and the substitute medical staff constitute the medical support team. When the primary care physician selects two or more medical support staffs, the primary care physician and the selected medical support staffs constitute the medical support team. If one of the medical support staffs declines, the primary care physician, the at least one medical support staff, and the substitute medical staff constitute the medical support team.

Associated data of an opinion includes the time of inputting the opinion and the name of the speaker of the opinion. The name of the speaker is identified by identifying the external support terminal 13 or the external substitute terminal 14, from which the opinion is transmitted, based on the data received along with the opinion. Then the name of the medical support staff using the external support terminal 13 or the external substitute terminal 14 identified is retrieved from the medical support staff database 25a. The name of the medical support staff retrieved is the name of the speaker of the opinion. The associated data of the examination image includes the examination image ID, the time of imaging, and the modality used. Note that the examination image is reduced to an appropriate size before the delivery.

A delivery information database 27a is connected to the delivery server 27. Various types of medical information, delivered or distributed to each of the external support terminal 13 and/or the external substitute terminal 14 and the in-hospital terminal 16 while the medical support is performed and the medical support request data are stored in the delivery information database 27a as a delivery data record. The delivery data record includes a registration ID, a delivery ID for identifying the delivered data, a delivery time, a time of receiving the data (an opinion or an examination image) which is the source of the delivered data, an ID (the primary care physician's ID or a medical support staff's ID) of a speaker who inputted an opinion, and content of the delivered data. When the delivered data is an opinion, the content of the opinion is stored in the delivery data record. When the delivered data is an examination image, its examination image ID and a device ID of the modality 23 used for imaging the examination image are stored in the delivery data record.

When the above-described substitute medical staff starts the medical support, the delivery server 27 sends all the medical information, delivered or distributed before the start of the medical support of the substitute medical staff, to the external substitute terminal 14.

The terminal control server 26 controls the external support terminal 13 and the external substitute terminal 14 through a support application. The terminal control server 26 deletes the delivered data stored in the external support terminal 13 and/or the external substitute terminal 14 when a case of the medical support is concluded, for example. When the medical support staff stops the medical support, the terminal control server 26 deletes the delivered data from the external support terminal 13 used by the decliner, being the medical support staff who stopped the medical support.

Similar to the in-hospital terminal 16, the external support terminal 13 is composed of a personal computer. For example, an application exclusive for a medical support system is installed on the external support terminal 13. The external support terminal 13 comprises a monitor 13a on which various screens pertaining to the medical support are displayed. The external support terminal 13 comprises a keyboard 13b and a mouse (not shown) with which various instructions pertaining to the medical support and text or letters are inputted.

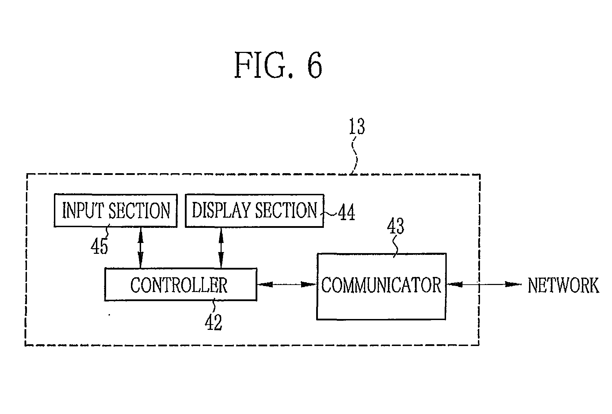

As shown in Fig. 6, the external support terminal 13 functions as a controller 42, a communicator 43, a display section 44, and an input section 45 when the installed support application is executed. The controller 42 controls the communicator 43, the display section 44, and the input section 45. The communicator 43 is composed of a communication circuit such as a network interface. The communicator 43 communicates with the medical support apparatus 17 through the network 12 and the firewall 21. The display section 44 comprises the monitor 13a and the like and displays a medical support request receiving screen and the like. The input section 45 is composed of the keyboard 13b. The input operation using the input section 45 allows the participation in the medical support, declining the medical support request, stopping or discontinuing the medical support, inputting an opinion during the medical support, access to an examination image, and the like.

The external substitute terminal 14 is an external support terminal used by a substitute medical staff. The configuration of the external substitute terminal 14 is the same as that of the external support terminal 13. A detailed description of the external substitute terminal 14 is omitted.

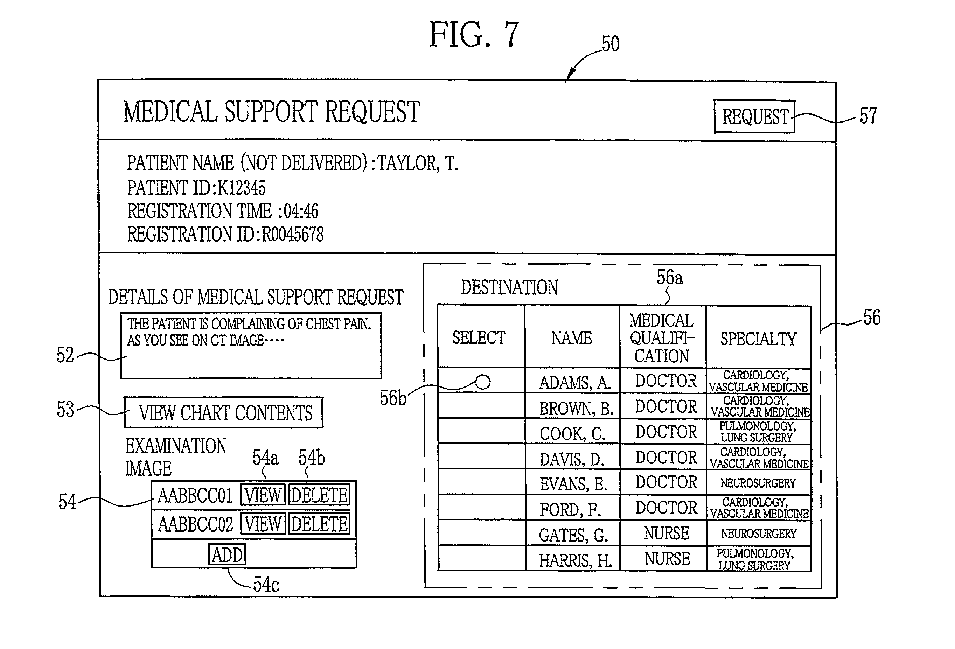

Next, screens of the in-hospital terminal 16 and the external support terminal 13 displayed at various steps of the medical support are described. In Fig. 7, a medical support request screen 50 is displayed on the monitor 16a of the in-hospital terminal 16. The medical support request screen 50 is displayed when a predetermined operation is performed on an electronic chart screen of the in-hospital terminal 16, for example. A medical support request command based on the input and setting on the medical support request screen 50 is sent from the in-hospital terminal 16 to the medical support apparatus 17.

The medical support request screen 50 displays a part of the contents of the electronic chart of the patient, being the subject of the medical support. The patient name, the patient ID, the registration time, and the registration ID displayed on the medical support request screen 50 are the same as those displayed on the electronic chart screen that displays the electronic chart of the case of the patient. Note that the patient ID, the registration time, and the registration ID may be transmitted with the medical support request from the delivery server 27 to the external support terminal 13. Transmission or distribution of the patient name may be prevented because the patient name is information to identify an individual.

The primary care physician inputs the medical support request comment to a comment box 52. The medical support request comment is, for example, a comment describing a reason for the medical support request. By clicking a chart content button 53 with the mouse, the in-hospital terminal 16 displays the contents of the electronic chart to be transmitted with the medical support request, for example, a symptom, the primary care physician's findings, a treatment history, and the like, out of all the contents of the electronic chart.

The medical support request screen 50 displays an examination image list 54. The examination image list 54 is a list of examination image IDs of the examination images to be transmitted by the delivery server 27. The delivery server 27 transmits the examination image list 54 together with the medical support request. When the medical support request screen 50 is initiated, the in-hospital terminal 16 automatically accesses the examination image server 19, using the registration ID as a key. The in-hospital terminal 16 retrieves the examination image IDs of the examination images, pertaining to the medical support request, taken between the acceptance of the patient and the startup of the medical support request screen 50. The retrieved examination image IDs are displayed on the examination image list 54.

A view button 54a and a delete button 54b are displayed for each examination image ID on the examination image list 54. With a click on the view button 54a, the in-hospital terminal 16 retrieves the corresponding examination image from the examination image server 19 and displays it on the display. With a click on the delete button 54b, the corresponding examination image ID is deleted from the examination image list 54. Thereby, an examination image unnecessary for the medical support is removed from the examination image list 54.

An add button 54c is displayed on the examination image list 54. The add button 54c is used to add an examination image to be transmitted. With a click on the add button 54c, the in-hospital terminal 16 displays a screen (selection screen) for selecting examination image(s) pertaining to the case of the patient, being the subject of the medical support. The examination image selected on the selection screen is added as the examination image to be transmitted. The examination image ID of the selected examination image is added to the examination image list 54. Thereby, the examination images pertaining to the medical support, out of all the examination images of the patient, are transmitted.

The medical support request screen 50 displays a destination selection section 56 on which a medical support staff, to whom the medical support is requested, is appointed or selected. The destination selection section 56 displays a list 56a of names, medical qualifications or job titles, and specialties of the medical support staffs retrieved from the medical support staff database 25a. The primary care physician uses the mouse or the like to select a medical support staff from the list 56a. A mark 56b is displayed for the medical support staff selected. Note that the name of the selected medical support staff may be highlighted or blinked instead of displaying the mark 56b. If the medical support relates to two or more diseases, two or more doctors of different specialties are selected or appointed. It is also possible to select or appoint two or more doctors of the same specialty.

In response to a click on a request send button 57, the in-hospital terminal 16 sends the medical support request command to the medical support apparatus 17. The medical support request command includes the data inputted and set on the medical support request screen 50. In the medical support apparatus 17, the data of the selected destination (the selected medical support staff) is inputted to the management server 25. The registration ID, the medical support request comment, and the examination image ID(s) selected from the examination image list 54 are inputted to the delivery server 27. Based on the registration ID and the examination image ID(s), pieces of data to be transmitted as the medical support request data are retrieved from the data servers.

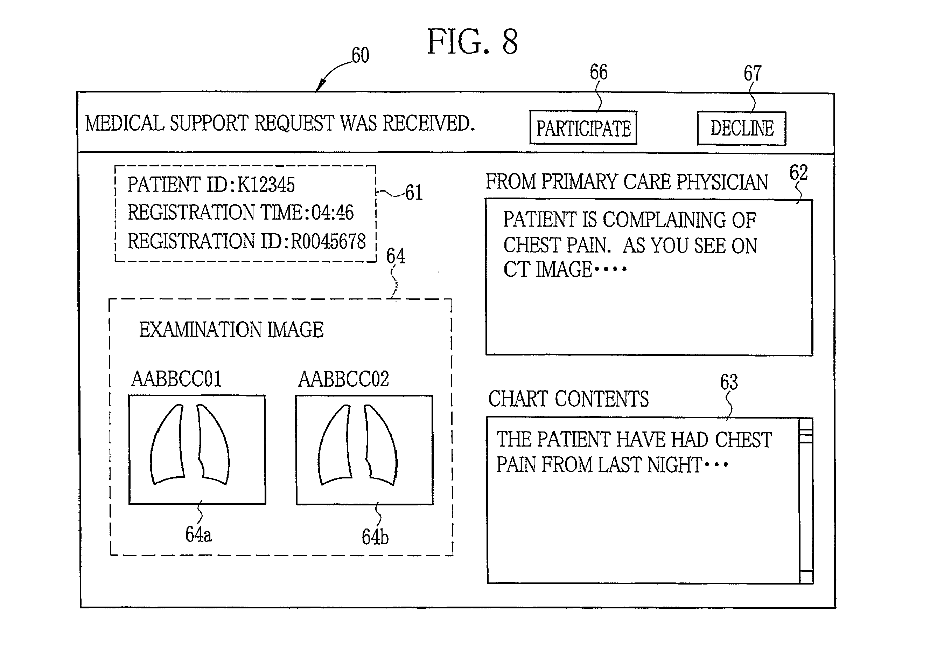

The external support terminal 13 receives the medical support request from the delivery server 27. As shown in Fig. 8, the display section 44 displays a medical support request receiving screen 60 on the monitor 13a, by way of example. The medical support request receiving screen 60 displays identification information 61, a medical support request comment 62 inputted to the comment box 52, chart contents 63 of the electronic chart pertaining to the case, and an examination image display section 64. The identification information 61 includes the patient ID, the registration time, the registration ID, and the like delivered or distributed as the medical support request data from the delivery server 27. The examination image display section 64 displays reduced images 64a and 64b corresponding to the examination image IDs in the examination image list 54. By clicking on the reduced image 64a or 64b with the mouse, the selected reduced image is enlarged and displayed as the examination image.

A participate button 66 and a decline button 67 are displayed on the medical support request receiving screen 60. With a click on the participate button 66, a participation notification is sent from the external support terminal 13 to the medical support apparatus 17. With a click on the decline button 67, a decline notification is sent from the external support terminal 13 to the medical support apparatus 17.

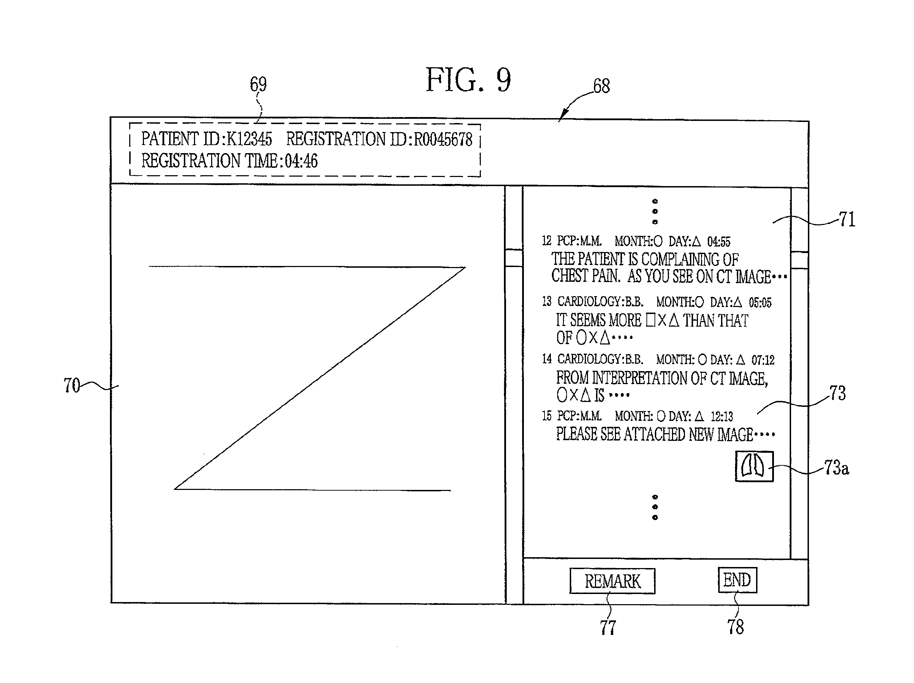

As shown in Fig. 9, when the medical support performed by the medical support team is started, the displays section 44 displays a medical support screen 68 on the monitor 13a. The medical support screen 68 is provided with an identification display section 69, a chart display section 70, and a remark display section 71. The identification display section 69 displays the identification information 61 such as the patient ID, the registration time, and the registration ID. The chart display section 70 displays the contents of the electronic chart. When all of the contents of the electronic chart concerned cannot be displayed within a display area of the chart display section 70, a slider 70a is displayed on a side of the chart display section 70. The slider 70a is operated to scroll the chart display section 70.

The remark display section 71 displays the comment inputted to the comment box 52 of the medical support request screen 50 and opinions sent from the in-hospital terminal 16 and opinions sent from the external support terminal 13 or the external substitute terminal 14. The comment and opinions are displayed in time order. Each remark or opinion 73 displayed on the remark display section 71 comprises the name of the speaker, the time of inputting the opinion, and the content of the opinion. The opinions 73 on the remark display section 71 provide a treatment history of the patient.

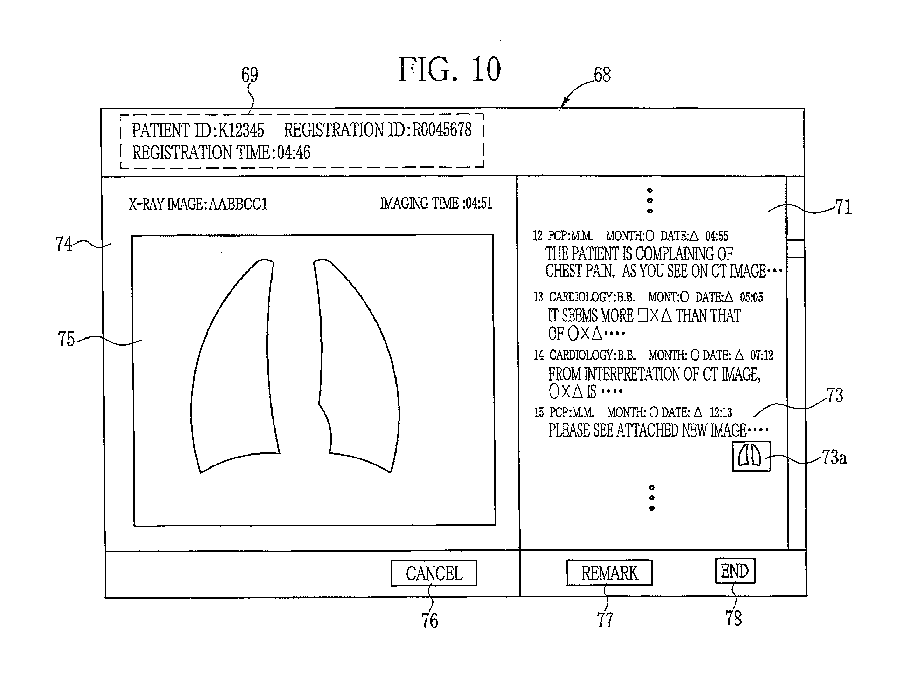

A reduced image 73a of an examination image is displayed near the opinion 73 when the examination image is transmitted together with the opinion 73. As shown in Fig. 10, in response to a click on the reduced image 73a with the mouse, the chart display section 70 switches to an examination image display section 74. A full-size examination image 75 of the reduced image 73a is displayed together with the type of the examination image (for example, an X-ray image or a CT image), the examination image ID, and the imaging time. A medical support staff observes details of the examination image 75 on the examination image display section 74. Note that a tool bar is displayed when a CT image, an MRI image, or the like is displayed. The tool bar is used to control a cross-sectional view and an orientation of the examination image displayed. The tool bar allows observation of a desired cross-sectional view of the examination image 75 in a desired direction. A cancel button 76 is located in a lower portion the examination image display section 74. The examination image display section 74 returns to the chart display section 70 when the cancel button 76 is clicked.

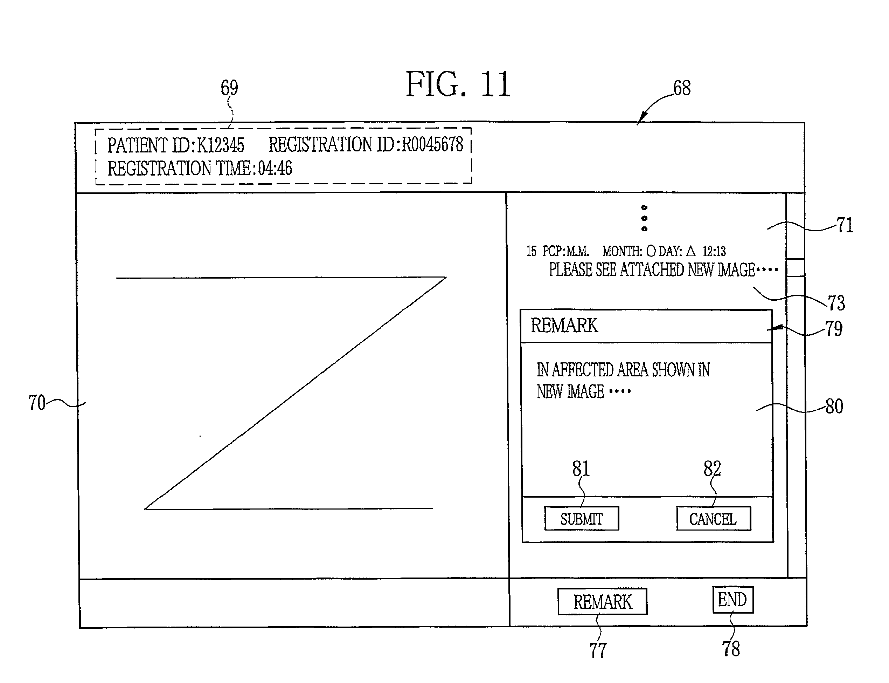

A remark button 77 and an end button 78 are displayed in the lower portion of the remark display section 71. As shown in Fig. 11, in response to a click on the remark button 77, the display section 44 displays a remark input section 79 on the remark display section 71. The remark input section 79 is provided with a remark box 80, a submit button 81, and a cancel button 82. A remark or opinion is inputted to the remark box 80 through the keyboard or the like. In response to a click on the submit button 81, the controller 42 sends the opinion, inputted to the remark box 80, to the medical support apparatus 17 through the communicator 43. The delivery server 27 receives the opinion and delivers or distributes the opinion to each of the in-hospital terminal 16 and the external support terminal 13. The cancel button 82 is used to close the remark input section 79 to return to the screen displayed before the remark input section 79.

In Fig. 9, the end button 78 is operated when the medical support staff stops the medical support. In response to this, the controller 42 sends the decline notification to the medical support apparatus 17. Based on the decline notification, the medical support apparatus 17 requests the medical support staff to select or recommend a substitute medical staff to replace him/her.

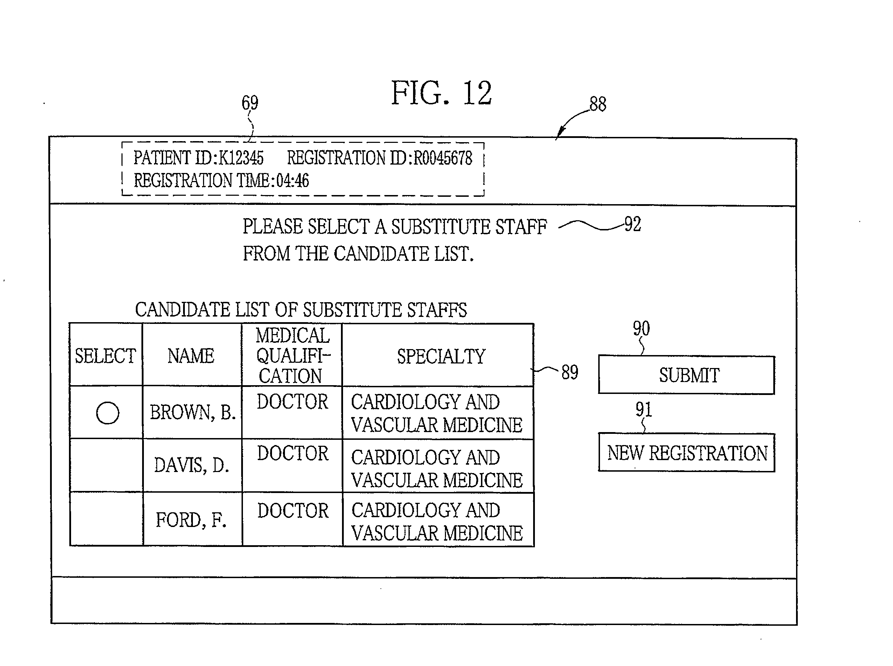

A substitute medical staff selection screen 88 (see Fig. 12) is displayed on the monitor 13a of the external support terminal 13 when the medical support apparatus 17 sends the substitute medical staff request and a candidate list of the substitute medical staffs to the external support terminal 13. The substitute medical staff selection screen 88 displays the identification display section 69, a candidate list 89, a submit button 90 and a new registration button 91. The candidate list 89 displays the names, the medical qualifications or job titles, and the specialties of the medical support staffs retrieved by the substitute medical staff requesting section 36. The candidate list 89 is a list of candidates for the substitute medical staff with the same medical qualification and the same specialty as those of the decliner (in this example, "ADAMS, A."). The candidates in the candidate list 89 are retrieved from the candidate list 56a shown in Fig. 7. A message 92 is displayed above the candidate list 89. The message 92 prompts the medical support staff (decliner) to select a substitute medical staff from the candidate list 89 and transmit the selection result. The decliner selects a substitute medical staff from the candidate list 89 with the mouse and then clicks on the submit button 90. Thereby, information pertaining to the selected substitute medical staff is transmitted to the medical support apparatus 17.

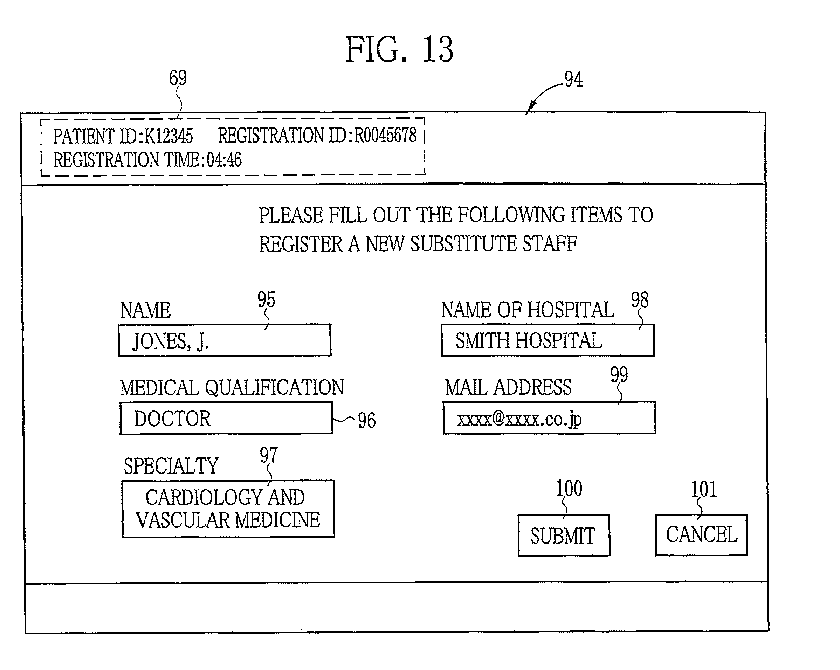

The new registration button 91 is operated to register a new substitute medical staff. A new substitute medical staff registration screen 94 (see Fig. 13) is displayed on the monitor 13a of the external support terminal 13 when the new registration button 91 is clicked. The new substitute medical staff registration screen 94 is provided with the identification display section 69, input boxes 95 to 99, a submit button 100, and a cancel button 101. The name, the medical qualification or job title, and the specialty of the new substitute medical staff, the name of the medical institution to which the new substitute medical staff belongs, the mail address of the new substitute medical staff, and the like are inputted to the input boxes 95 to 99. Then, the submit button 100 is clicked to transmit the input to the medical support apparatus 17. The cancel button 101 is clicked to decline the registration (namely, to decline joining the medical support team) of a new substitute medical staff.

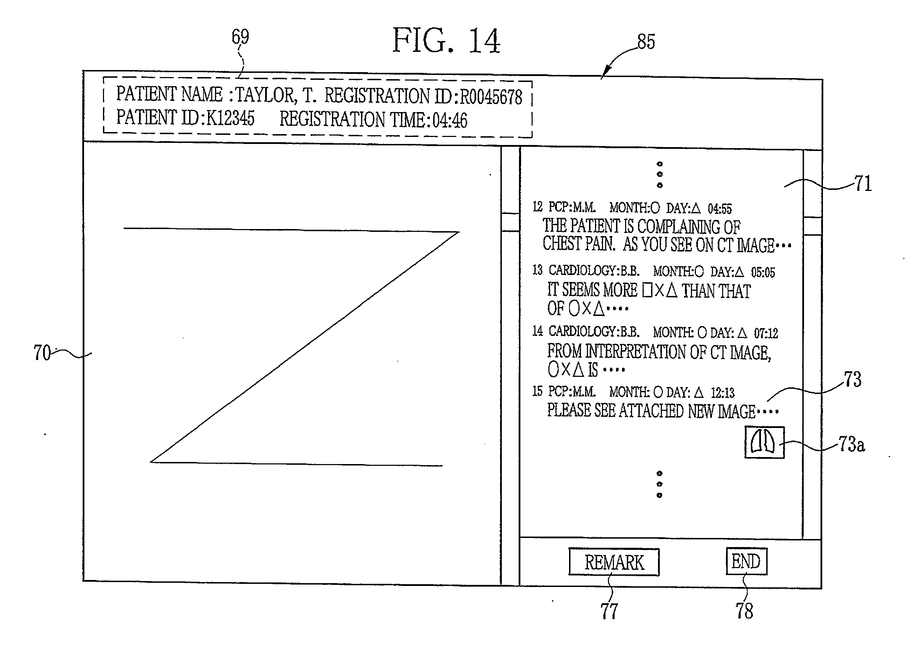

When or after the medical support is started, a medical support screen 85 (see Fig. 14) is displayed on the monitor 16a of the primary care physician's in-hospital terminal 16. A configuration of the medical support screen 85 is substantially the same as that of the medical support screen 68 in Fig. 9. The medical support screen 85 of the in-hospital terminal 16 differs from the medical support screen 68 of the external support terminal 13 in that the identification display section 69 of the medical support screen 85 displays the name of the patient. By clicking on the reduced image 73a on the medical support screen 85, the examination image display section 74 is displayed, in a manner similar to that in the medical support screen 68. By clicking on the remark button 77 on the medical support screen 85, the remark input section 79 is displayed, in a manner similar to that in the medical support screen 68. In response to a click on the end button 78, the medical support is concluded after a predetermined end sequence. A request for a substitute medical staff is not made when the medical support is ended by operating the in-hospital terminal 16.

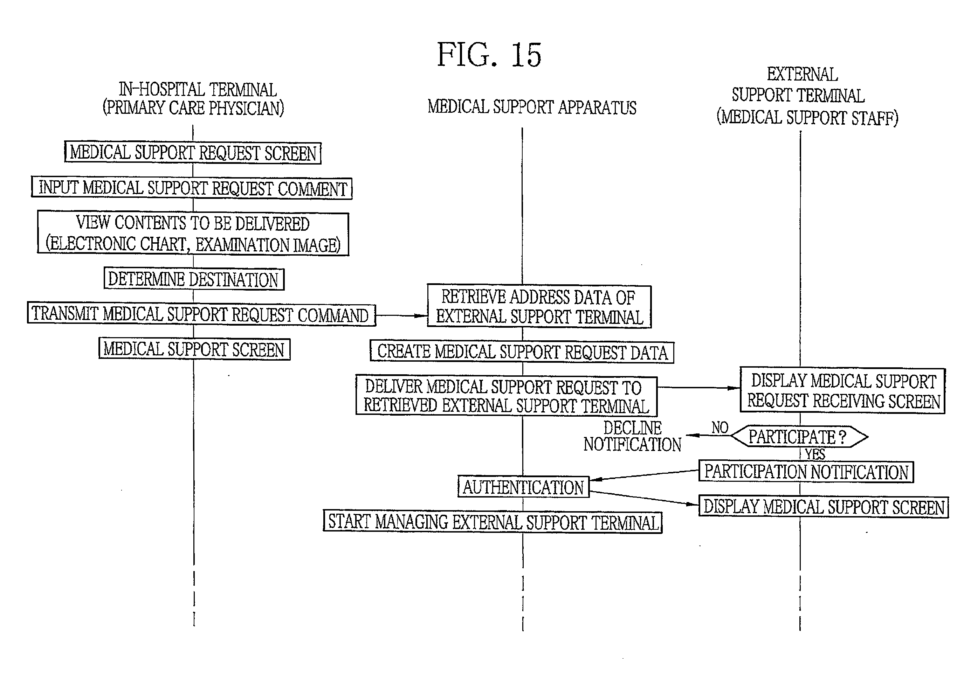

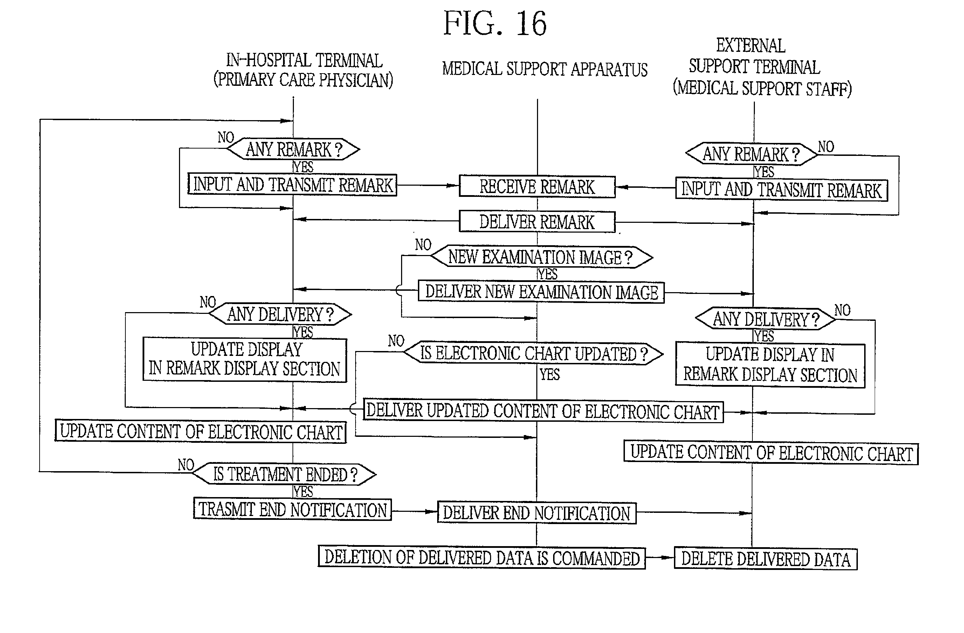

Referring to Figs. 15 and 16, an operation of the above configuration is described. The medical support system 10 is used when a doctor or primary care physician needs advice from a specialist or the like on treatment of a patient. A patient may be an outpatient, an emergency outpatient in out-of-office hours, an emergency patient delivered by ambulance, an inpatient, or the like.

When the medical support is necessary for the treatment (which may be referred to as the case) of the patient currently treated, the primary care physician operates the in-hospital terminal 16 to display the electronic chart screen corresponding to the patient. Then the primary care physician performs a predetermined operation on the electronic chart screen to display the medical support request screen 50 shown in Fig. 7. In the medical support request screen 50, the patient ID and the registration ID are retrieved from the electronic chart screen.

After the medical support request screen 50 is displayed, the primary care physician inputs a medical support request comment to the comment box 52. The medical support request comment includes, for example, a reason for the medical support request or the content of the advice the primary care physician needs. The primary care physician clicks on the chart content button 53 to check the contents, of the electronic chart of the case, to be transmitted. The in-hospital terminal 16 retrieves the contents of the electronic chart from the electronic chart server 18, using the registration ID as the key. The in-hospital terminal 16 displays the contents retrieved. If necessary, the primary care physician adds content to the electronic chart or inputs the content as a medical support request comment to the comment box 52, using the in-hospital terminal 16.

The examination image IDs of the examination images already taken are retrieved from the electronic chart and displayed on the examination image list 54. To check or view an examination image, the primary care physician clicks on the corresponding view button 54a. In response to this, the in-hospital terminal 16 retrieves the examination image having the examination image ID corresponding to the view button 54a from the examination image server 19 and displays the examination image retrieved. If the displayed examination image is unnecessary or unrelated to the case, the examination image is closed and then the delete button 54b corresponding to the examination image ID of the examination image is clicked. Thereby, the examination image ID is deleted from the examination image list 54. Note that, if the examination image is to be transmitted, no additional operation is necessary after the examination image is closed.

The primary care physician clicks on the add button 54c when he/she needs to additionally deliver an examination image taken during the past consultation, for example. In response to this, the in-hospital terminal 16 displays a selection screen. The selection screen displays, for example, thumbnail images of the examination images of the patient registered in the examination image database 19a. The primary care physician selects a necessary examination image from the thumbnail images. When the examination image is selected, an examination ID of the selected examination image is displayed on the examination image list 54. The above-described operation is repeated to add two or more examination images.

The primary care physician refers to the candidate list 56a and selects a medical support staff (destination) in the destination selection section 56 in accordance with the content of the medical support, for example. Then, the primary care physician clicks on the request send button 57. In response to this, the medical support request command is sent from the in-hospital terminal 16 to the medical support apparatus 17. The medical support request command is made based on the contents inputted and set on the medical support request screen 50. The medical support request command has command data including the patient ID, the registration time, the registration ID, the medical support request comment, the examination image IDs displayed on the examination image list 54. The name of the primary care physician who sent the medical support request command is transmitted together with the medical support request command. Thereafter, the medical support screen 85 is displayed on the in-hospital terminal 16 as shown in Fig. 14.

When the medical support request receiving section 30 of the management server 25 receives the medical support request command, the address retrieving section 31 accesses the medical support staff database 25a. The address retrieving section 31 goes through the medical support staff records and retrieves the address data, stored in the medical support staff record, corresponding to the selected medical support staff. For example, when "Adams, A." is selected as the medical support staff in the destination selection section 56 shown in Fig. 7, the address data of the external support terminal 13 used by "Adams, A." is retrieved from the medical support staff database 25a in Fig. 2. The address data is sent as the destination to the delivery server 27.

The registration ID and the medical support request comment, out of the command data, and the examination image ID selected from the examination image list 54 are sent to the delivery server 27. The delivery server 27 retrieves the contents of the electronic chart corresponding to the registration ID from the electronic chart server 18. The delivery server 27 retrieves the examination image corresponding to the examination image ID from the examination image server 19. The delivery server 27 creates the medical support request data from the contents of the electronic chart, the examination image, the patient ID, the registration time, the registration ID, the examination image ID, the medical support request comment, and the name of the primary care physician. The medical support request including the medical support request data is transmitted from the delivery server 27.

The medical support request, including the medical support request data, is transmitted from the delivery server 27 to the address shown in the address data retrieved by the management server 25, through the firewall 21 and the network 12. Namely, the medical support request is transmitted to the external support terminal 13 of the medical support staff selected in the destination selection section 56 of the in-hospital terminal 16. The content of the medical support request including the medical support request data is stored as the delivery data record.

A part of functions of the external support terminal 13 is kept turned on to receive the medical support request. When the external support terminal 13 of the selected medical support staff receives the medical support request, the external support terminal 13 displays the medical support request receiving screen 60 shown in Fig. 8.

When the external support terminal 13 displays the medical support request receiving screen 60, the medical support staff using the external support terminal 13 refers to the medical support request comment 62 and the chart contents 63 displayed in the medical support request receiving screen 60. The medical support staff clicks on the reduced images 64a and 64b in the examination image display section 64 and views the examination images when necessary. The medical support staff determines whether to participate in the medical support according to his/her schedule or the like. The medical support staff operates the participate button 66 when he/she participates in the medical support and operates the decline button 67 when he/she declines the medical support request. For example, when the medical support staff determines to decline the medical support request, he/she operates or clicks on the decline button 67. In response to this, the decline notification is transmitted from the external support terminal 13 to the medical support apparatus 17. After that, the opinions and the like pertaining to the medical support are not transmitted to this external support terminal 13 and the medical support request receiving screen 60 is closed.

The medical support staff operates or clicks on the participate button 66 to participate in the medical support. In response to this, the monitor 13a displays an input screen for authentication ID and password. The medical support staff inputs the predetermined authentication ID and the password using the keyboard and then operates or clicks on the submit button (not shown).

In response to operating the submit button, the participation notification, together with the authentication ID and the password, are sent from the external support terminal 13 to the medical support apparatus 17 through the network 12 and the firewall 21.

The participant data receiving section 32 of the management server 25 receives the authentication ID and the password sent from the external support terminal 13. From the information obtained at the reception of the authentication ID and the password, the participant data receiving section 32 identifies the external support terminal 13 which transmitted the authentication ID and the password. The participant data receiving section 32 retrieves the medical support staff record, in which the terminal data corresponding to the identified external support terminal 13 is stored, from the medical support staff database 25a. The participant data receiving section 32 checks whether the authentication ID and the password stored in the medical support staff record match those sent from the external support terminal 13.

For example, when the authentication ID or the password does not match, the participant data receiving section 32 transmits a message to the external support terminal 13 through the delivery server 27. The message notifies that the authentication ID or the password is incorrect and prompts re-entering the authentication ID or the password. Note that when the participant data receiving section 32 cannot find the medical support staff record with the terminal data which corresponds to the external support terminal 13, the participant data receiving section 32 allows the external support terminal 13 to display a message notifying that the external support terminal 13 has not been registered.

When the authentication ID and the password match those in the medical support staff record, the participant data receiving section 32 determines that the medical support staff using the external support terminal 13 is valid. The terminal management section 33 manages the information pertaining to the external support terminal 13 of the authenticated medical support staff, namely, the terminal management section 33 manages the external support terminal 13 as the terminal through which the medical support is provided to the in-hospital terminal 16.

The delivery server 27 sends an authentication notification to the external support terminal 13 when the terminal management section 33 starts managing the external support terminal 13. The authentication notification notifies that the authentication is completed properly. In response to receiving the authentication notification, the monitor 13a of the external support terminal 13 displays the medical support screen 68 in Fig. 9. If the primary care physician and the medical support staff have not inputted any remark or opinion, only the medical support request comment and the examination image are displayed on the remark display section 71 of each of the medical support screen 68 shown in Fig. 9 and the medical support screen 85 shown in Fig. 14. Thereby, the medical support team is formed. The terminals used by the respective members of the team are managed as a group. The medical information is transmitted and received within the terminal group.

To input a remark or opinion, the primary care physician or the medical support staff operates or clicks on the remark button 77 of the medical support screen 68 or 85. In response to this, the remark input section 79 is displayed as shown in Fig. 11. After a remark or opinion such as advice or findings are inputted to the remark box 80, the submit button 81 is clicked. Thereby, the opinion inputted to the remark box 80 is transmitted to the medical support apparatus 17.

Note that the chart display section 70 shown in Fig. 9 allows viewing the contents of the electronic chart. To view an examination image, the medical support staff clicks on the reduced image 73a displayed on the remark display section 71. Thereby, the chart display section 70 is switched to the examination image display section 74 as shown in Fig. 10.

The delivery server 27 receives the opinions sent from the external support terminal 13 and the in-hospital terminal 16. The delivery server 27 delivers the received opinions to the external support terminal 13 and the in-hospital terminal 16. The delivered opinions are stored as the delivery data record in the delivery information database 27a. When the external support terminal 13 and the in-hospital terminal 16 receive the opinions from the delivery server 27, the content of the remark display section 71 is updated in each of the medical support screen 68 of the external support terminal 13 and the medical support screen 85 of the in-hospital terminal 16.

When an image of the patient, being the subject of the medical support, is taken with the modality 23, the examination image server 19 checks the patient ID and then sends the taken examination image to the delivery server 27. The delivery server 27 delivers the examination image to the external support terminal 13 and the in-hospital terminal 16. Thereby, the remark display section 71 of each of the external support terminal 13 and the in-hospital terminal 16 displays the reduced image 73a of the examination image delivered. In response to a click on the reduced image 73a, the chart display section 70 is switched to the examination image display section 74 displaying the examination image 75. An examination image is transmitted and its reduced image is displayed in the remark display section 71 in a manner similar to the above every time an image of the patient, being the subject of the medical support, is taken with the modality 23.

When the primary care physician provides input to an electronic chart through the in-hospital terminal 16, the electronic chart server 18 adds the content of the input to the treatment data record in the electronic chart database 18a. Based on the registration ID, the electronic chart server 18 detects that the treatment data record to which the content is added pertains to the case the patient, being the subject of the medical support, and sends the added content to the delivery server 27. The delivery server 27 delivers the added content to each of the external support terminal 13 and the in-hospital terminal 16. The contents of the electronic chart including the added content are displayed on the chart display section 70.

During the diagnosis and treatment performed by the medical support team, the primary care physician and the medical support staff consult with each other and the medical support staff gives the primary care physician advice while they refer to the opinions and the examination image displayed in the remark display section 71. The medical support thus allows the primary care physician to diagnose and treat a disease or injury appropriately even if it is out of his/her specialty.

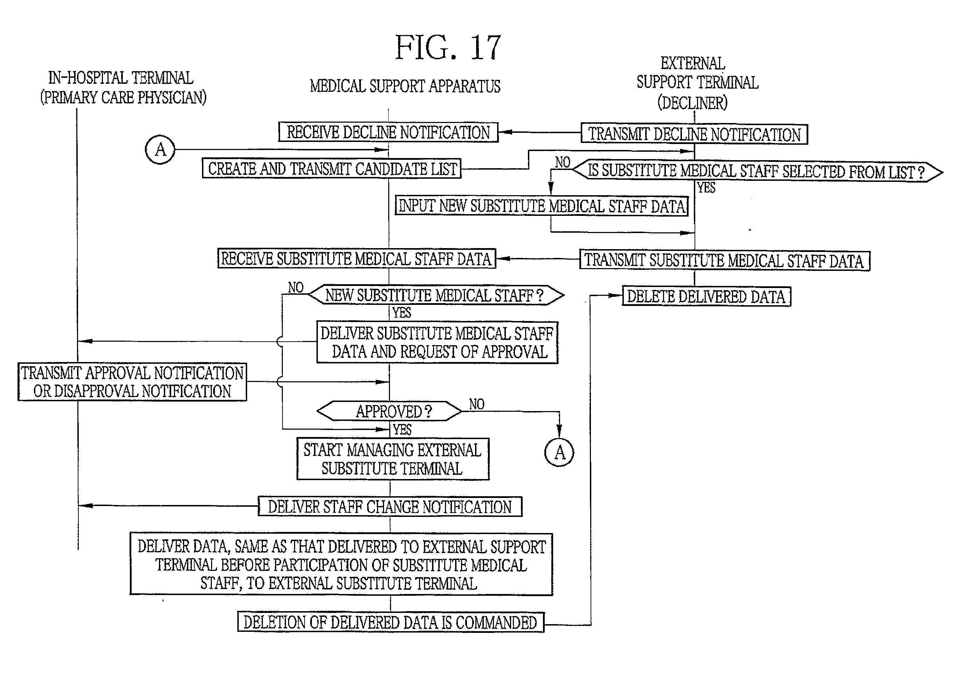

Next, referring to Fig. 17, an operation performed by the medical support staff to decline or stop participating in the medical support is described. To decline or stop participating in the medical support, the medical support staff clicks on the end button 78 shown in Fig. 9. In response to this, the external support terminal 13 sends the decline notification to the medical support apparatus 17. The decline notification receiving section 35 of the management server 25 receives the decline notification. The substitute medical staff requesting section 36 accesses the medical support staff database 25a and retrieves the medical support staff with the same medical qualification (or job title) and the same specialty as those of the medical support staff (decliner) who declines or stops participating in the medical support. The substitute medical staff requesting section 36 creates the candidate list of the substitute medical staffs who are capable of providing the medical support in place of the medical support staff (decliner). For example, when the name of the medical support staff (decliner) is "Adams, A." registered in the medical support staff database 25a in Fig. 2, the substitute medical staff requesting section 36 retrieves "Brown, B.", "Davis, D.", and "FORD, F." with the same medical qualification or job title ("doctor") and the same specialty ("cardiology and vascular medicine") as those of "Adams, A." to create the candidate list.

The substitute medical staff requesting section 36 transmits the candidate list and the substitute medical staff request to the external support terminal 13. As shown in Fig. 12, the monitor 13a of the external support terminal 13 displays the substitute medical staff selection screen 88. The substitute medical staff selection screen 88 displays the candidate list 89 of the substitute medical staffs. The medical support staff (decliner) selects one of the substitute medical staffs from the candidate list 89 and then clicks on the submit button 90. The substitute medical staff data receiving section 37 of the management server 25 receives the data or information of the selected substitute medical staff and the data or information of the substitute medical staff's external substitute terminal 14. The terminal management section 33 manages the external substitute terminal 14 as the external support terminal used for the medical support. The external substitute terminal 14 joins the medical support team in place of the external medical support terminal 13 of the medical support staff (decliner). Thereafter, the external substitute terminal 14, being a member of the medical support team, receives the medical information and is allowed to input an opinion pertaining to the medical support, for example.

The medical support staff (decliner) may register a new substitute medical staff that is not on the candidate list. To register a new substitute medical staff, the decliner clicks on the new registration button 91 on the substitute medical staff selection screen 88. In response to this, the external support terminal 13 displays the new substitute medical staff registration screen 94 shown in Fig. 13. The decliner inputs data of a new substitute medical staff on the new substitute medical staff registration screen 94 and then operates or clicks on the submit button 100. The substitute medical staff data receiving section 37 receives the data or information of the new substitute medical staff.

The substitute medical staff data receiving section 37 transmits the data of the new substitute medical staff and a request of approval to the in-hospital terminal 16. The primary care physician examines the data of the new substitute medical staff to determine whether to approve the participation of the new substitute medical staff. To approve, the primary care physician transmits a notification of approval (approval notification) from the in-hospital terminal 16 to the substitute medical staff data receiving section 37. To disapprove, the primary care physician transmits a notification of disapproval (disapproval notification) from the in-hospital terminal 16 to the substitute medical staff data receiving section 37. The substitute medical staff data receiving section 37 determines that the new substitute medical staff is approved when the approval notification is transmitted from the primary care physician. In response to this, the substitute medical staff data receiving section 37 allows the terminal management section 33 to start managing the substitute medical staff's external substitute terminal 14.

After starting the management of the external substitute terminal 14, the terminal management section 33 transmits the staff change notification to the in-hospital terminal 16. The data pertaining to the medical support transmitted before the participation of the new substitute medical staff is transmitted to the external substitute terminal 14. The delivered data pertaining to the medical support, stored in the external support terminal 13, is deleted under control of the terminal control server 26 through the network 12. Then, the primary care physician and the substitute medical staff proceed with the medical support. If the substitute medical staff uses the external support terminal 13, used by the medical support staff (decliner), as the external substitute terminal 14, the delivered data is used without deletion.

Note that the substitute medical staff data receiving section 37 determines that the new substitute medical staff is disapproved when the disapproval notification is transmitted from the primary care physician. The terminal management section 33 does not register the new substitute medical staff's external substitute terminal 14. In this case, the medical support apparatus 17 transmits the substitute medical staff request and the candidate list for the substitute medical staff to the decliner's external support terminal 13 again.

The primary care physician operates or clicks on the end button 78 on the in-hospital terminal 16 to end the medical support when the treatment is completed with the sufficient medical support, for example. In response to this, an end notification is sent to the medical support apparatus 17. The delivery server 27 sends the end notification to each of the external support terminal 13 and the in-hospital terminal 16. The terminal control server 26 controls the external support terminal 13 through the network 12 to delete the delivered data pertaining to the medical support stored in the external support terminal 13.

According to this embodiment, a substitute medical staff is found immediately after the medical support staff declines the medical support request or stops the medical support, so that interruption in the treatment is prevented. A substitute medical staff is selected from the candidate list, so that the substitute medical staff is selected quickly. A new substitute medical staff is registered and permitted to participate in the medical support when there is no available substitute medical staff in the candidate list. Because a new substitute medical staff needs the primary care physician's approval to participate in the medical support, reliability of the substitute medical staff to the primary care physician is improved.

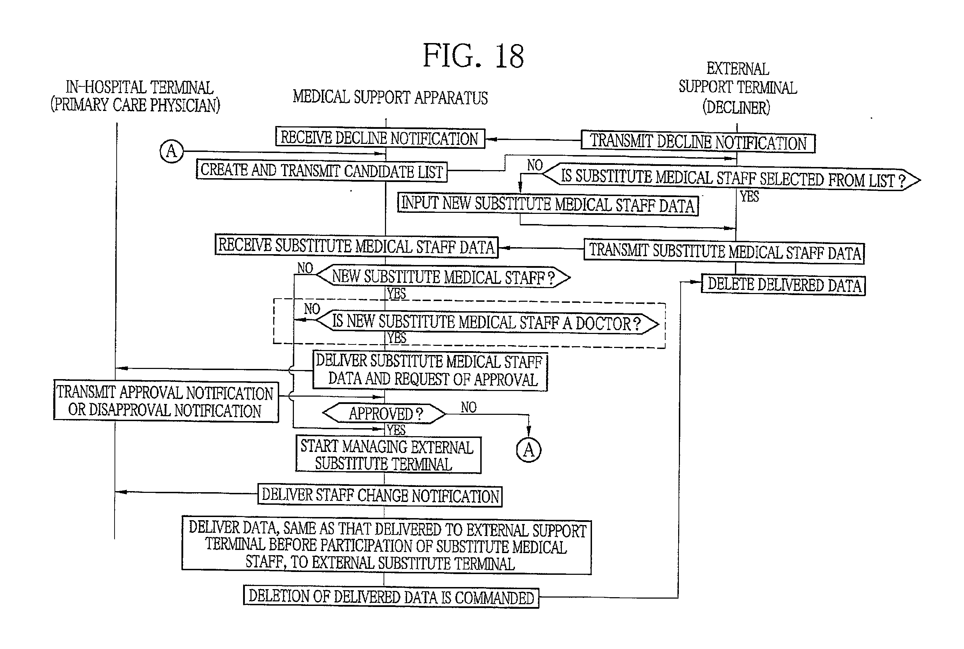

Next, a second embodiment is described. In the above embodiment, the primary care physician's approval is necessary before the registration of a new substitute medical staff. In this embodiment, the primary care physician's approval is unnecessary depending on the medical qualification or job title of the new substitute medical staff. For example, as shown in broken lines in Fig. 18, when a new substitute medical staff is a doctor, he/she is permitted to participate in the medical support only after the primary care physician' s approval because the doctor has broad responsibilities for the treatment. A nurse or a technologist is permitted to participate as a new substitute medical staff in the medical support without the primary care physician's approval because their responsibilities for the treatment are limited. Thereby, a substitute medical staff is registered quickly.

Various changes and modifications are possible in the present invention and may be understood to be within the present invention.

## Claims

1. A medical support apparatus (17) through which medical support is provided, the medical support apparatus being connected to an in-hospital terminal (16), at least one external support terminal (13), and at least one external substitute terminal (14) through a network (12), a primary care physician using the in-hospital terminal, the primary care physician requesting the medical support, a medical support staff using the at least one external support terminal, a substitute medical staff using the at least one external substitute terminal, the medical support apparatus comprising:
a delivery means (27) for delivering a medical support request to the each external support terminal, the medical support request being transmitted to the delivery means from the in-hospital terminal;
a decline notification receiving means (35) for receiving a decline notification for declining participation in the medical support, the decline notification being transmitted from a first external support terminal (13), the first external support terminal being one of the at least one external support terminal, a decliner that declines the medical support request using the first external support terminal;
a substitute medical staff requesting means (36) for transmitting a substitute medical staff request to the first external support terminal to request the decliner to recommend the substitute medical staff;
a substitute medical staff data receiving means (37) for receiving information of the substitute medical staff, recommended by the decliner, from the first external support terminal; and
a terminal management means (33) for managing the in-hospital terminal and the at least one external support terminal as a terminal group for performing the medical support, the terminal management means allowing a first external substitute terminal (14) used by the substitute medical staff, out of the at least one external substitute terminal, to join the terminal group in place of the first external support terminal when the substitute medical staff provides the medical support in place of the decliner, the delivery means delivering information pertaining to the medical support within the terminal group.

2. The medical support apparatus of claim 1, wherein the medical support staff is selected by the primary care physician from among candidates stored in a medical support staff candidate database (25a) and the medical support staff candidate database stores information pertaining to medical qualification and specialty of the candidates and information pertaining to the external support terminals.

3. The medical support apparatus of claim 2, wherein the substitute medical staff requesting means retrieves at least one of the candidates stored in the medical support staff candidate database to create a candidate list (89) for the substitute medical staff, and the retrieved candidate has same medical qualification and same specialty as those of the decliner, and the substitute medical staff requesting means transmits the candidate list and the substitute medical staff request to the first external support terminal, and the decliner uses the candidate list to recommend the substitute medical staff.

4. The medical support apparatus of one of claims 1 to 3, wherein the terminal management means notifies the primary care physician's in-hospital terminal that the medical support staff declines the participation in the medical support and the substitute medical staff participates in the medical support.

5. The medical support apparatus of claim 3 or 4, wherein when the decliner recommends an unregistered medical support staff, not listed in the candidate list, as the substitute medical staff, information of the unregistered medical support staff is transmitted from the first external support terminal to the substitute medical staff data receiving means.

6. The medical support apparatus of claim 5, wherein the substitute medical staff data receiving means transmits the information of the unregistered medical support staff to the in-hospital terminal to ask for primary care physician' s approval and permits the unregistered medical support staff as the substitute medical staff when the substitute medical staff data receiving section receives notification of the primary care physician's approval from the in-hospital terminal; and
the terminal management means manages the first external substitute terminal as one of terminals of the terminal group in place of the first external support terminal, and the first external substitute terminal is used by the substitute medical staff approved by the primary care physician.

7. The medical support apparatus of claim 5, wherein the substitute medical staff data receiving means permits the unregistered medical support staff as the substitute medical staff without primary care physician's approval when medical qualification of the unregistered medical support staff is other than a predetermined medical qualification; and
the terminal management means manages the first external substitute terminal as one of terminals of the terminal group in place of the fist external support terminal, and the first external substitute terminal is used by the substitute medical staff permitted by the substitute medical staff data receiving means.

8. A medical support method for providing medical support through a medical support system (10) in which an in-hospital terminal (16), at least one external support terminal (13), at least one external substitute terminal (14), and a medical support apparatus (17) being connected to each other through a network (12), a primary care physician using the in-hospital terminal, the primary care physician requesting the medical support, a medical support staff using the at least one external support terminal, a substitute medical staff using the at least one external substitute terminal, the medical support being provided through the medical support apparatus, the medical support method comprising the steps of:
(A) transmitting a medical support request from the in-hospital terminal to the medical support apparatus;
(B) delivering the medical support request from the medical support apparatus to the each external support terminal;
(C) transmitting a decline notification for declining participation in the medical support from a first external support terminal (13) to the medical support apparatus, the first external support terminal being one of the at least one external support terminal, a decliner that declines the medical support request using the first external support terminal;
(D) transmitting a substitute medical staff request from the medical support apparatus to the first external support terminal to request the decliner to recommend the substitute medical staff;
(E) transmitting information of the substitute medical staff recommended by the decliner from the first external support terminal to the medical support apparatus;
(F) managing the in-hospital terminal and the external support terminal as a terminal group for performing the medical support, a first external substitute terminal (14) used by the substitute medical staff, out of the at least one external substitute terminal, joining the terminal group in place of the first external support terminal when the substitute medical staff provides the medical support in place of the decliner, and
(G) delivering information pertaining to the medical support within the terminal group.

9. The medical support method of claim 8, wherein the primary care physician selects the medical support staff out of candidates stored in a medical support staff candidate database (25a) and the medical support staff candidate database stores information pertaining to medical qualification and specialty of the candidates and information pertaining to the external support terminals.

10. The medical support method of claim 9, wherein the step (D) further comprising the steps of:
(D1) retrieving at least one of the candidates stored in the medical support staff candidate database and creating a candidate list (89) for the substitute medical staff with the use of the medical support apparatus, the retrieved candidate having same medical qualification and same specialty as those of the decliner, the decliner using the candidate list to recommend the substitute medical staff; and
(D2) transmitting the substitute medical staff request and the candidate list from the medical support apparatus to the first external support terminal.

11. The medical support method of claim 10, wherein in the step (E), information of an unregistered medical support staff is transmitted from the first external support terminal to the medical support apparatus when the decliner recommends the unregistered medical staff, not on the candidate list, as the substitute medical staff.

12. The medical support method of claim 11, further comprising the steps of:
(H) transmitting the information of the unregistered medical support staff from the medical support apparatus to the in-hospital terminal to ask for primary care physician's approval; and
(I) permitting the unregistered medical support staff as the substitute medical staff by the medical support apparatus when the medical support apparatus receives notification of the primary care physician' s approval from the in-hospital terminal; and
wherein in the step (G), the first external substitute terminal used by the substitute medical staff approved by the primary care physician is managed as one of terminals of the terminal group in place of the first external support terminal.

13. The medical support method of claim 11, further comprising the step of:
(J) permitting the unregistered medical support staff as the substitute medical staff by the medical support apparatus without approval of the primary care physician when medical qualification of the unregistered medical staff is other than a predetermined medical qualification; and
wherein in the step (G), the first external substitute terminal used by the substitute medical staff permitted in the step (J) is managed as one of terminals of the terminal group in place of the first external support terminal.
